Europäisches Patentamt

European Patent Office

Office européen des brevets

(19)

(11) Publication number: **0 272 647**
**A2**

(12)

# EUROPEAN PATENT APPLICATION

(21) Application number: 87118839.7

(51) Int. Cl.⁴: **A61B 17/58**

(22) Date of filing: 18.12.87

(30) Priority: 19.12.86 PL 263198

(43) Date of publication of application:
29.06.88 Bulletin 88/26

(84) Designated Contracting States:
AT BE CH DE ES FR GB GR IT LI LU NL SE

(71) Applicant: Huta Baildon Przedsiebiorstwo
Panstwowe
ul. Zelazna 9
Katowice(PL)

(72) Inventor: Karas, Wlodzimierz
ul. 27-go stycznia 107/86
DabrowaGornicza(PL)
Inventor: Granowski, Robert, Dr. med.
ul. Swierczewskiego 71
Warszawa(PL)
Inventor: Tuziemsky, Aleksander
ul. Gospodarcza 19/801
Sosnowiec(PL)
Inventor: Cieplak, Jerzy
ul. Tiereszkowej 6
Dabrowa Gornicza(PL)
Inventor: Pilawski, Kazimierz
ul. Nowickiego 5/93
Warszawa(PL)
Inventor: Ramotowski, Witold, Prof. Dr. med.
ul. Irysowa 106
Warszawa(PL)

(74) Representative: Füchsle, Klaus, Dipl.-Ing. et al
Hoffmann . Eitle & Partner Patentanwälte
Arabellastrasse 4
D-8000 München 81(DE)

(54) Bone bolt nut wrench.

(57) The subject of this present invention is a bone bolt nut wrench for bone anastomosis by the plate method.

A wrench is characterized in that two holes have been drilled behind socket (2) made in the face of body (1) ground off on the external side, said two holes accommodating spring part (5) and nut (6) is placed behind holder running inside body (1) screwed with one of its ends onto adjustment screw (4) and with the other entering socket (2), said nut (6) adjoining press pad (7) introduced through a hole of body (1) into a hole of adjustment screw (4) and secured with screw (8).

## Bone bolt nut wrench

The subject of this present invention is a bone bolt nut wrench for the bone bolts used for the anastomosis of bones by the plate method.

A bone compression anastomosis set consists of a compression plate, bone bolts and bone nuts. The set is fitted in the manner that the bolts are introduced in the holes drilled into the bone and on the former is fitted the compression plate secured to the bone bolts with the bone nuts, appropriate wrenches being used for screwing in the nuts.

A face-grooved nut wrench is known from Utility Design Specification protected with right of protection No. 40,279. The wrench is characterised in that it has a body in the form of a bush terminating at one end in splines corresponding to the groove in the nut and at the other end in a handle. Inside the body there is an externally threaded spring sleeve slideably mounted from the side of the splines and pressed to the splines with a spring.

In addition to the above, it has a push pad mounted outside of the body and connected to the spring sleeve with a threaded pin screwed into the spring sleeve. Four splines are concentrically and uniformly spaced on the end face of the body. These splines terminate in points. The spring sleeve has at least two longitudinal cuts and a thread of incomplete profile.

The disadvantage of the wrench used up to now is its unreliability, since the force with which the nut can be screwed home is limited by the strength of the splines, any inclination of the wrench causing the splines to be disengaged from the grooves of the nut. The screw spring used as a hold-down part does not fully ensure the correct action of the wrench, the latter being used only for screwing the nuts on and off the bone bolts.

On the other hand, the bone bolts require the application of a special screw-driver. Thus, to mount or dismount the compression plate requires the use of two tools during an operative treatment, which poses additional difficulties.

The purpose of this present invention has been to develop a wrench of the design allowing it to be used both for screwing the nuts onto the bone bolts and screwing the bone bolts in and out.

The essence of the design as per this present invention is that there are two holes drilled behind the square socket made in the body face ground off at the external side and mounting a spring part. Inside the body there is a holder screwed on with its one end to an adjustment screw and, with the other end, entering the socket. A nut is provided behind the holder. The push pad fitted through hole in the body into that in the adjustment screw and placed behind the nut is screwed down with a bolt.

The wrench of the design as per this present invention can be used both for screwing on and down the bone bolts and for screwing the nuts onto these bone bolts, which eliminates the need for using an additional screw driver. The square socket and the holder ensure the sure application of the wrench, both on the bolt head and on the nut. This eliminates the danger of damaging the wrench and its slipping during its application. The holder provided in the working end of the wrench allows the bolt or the nut to be applied earlier, which markedly facilitates handling these minute parts during an operative treatment.

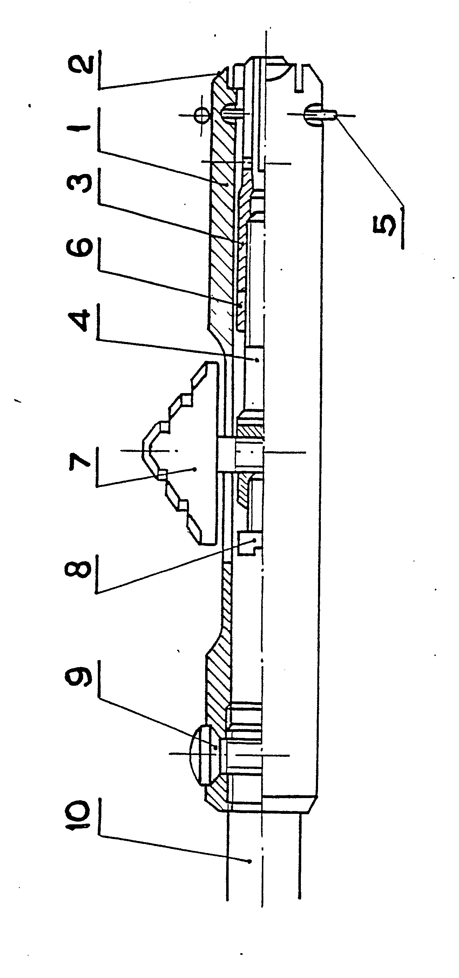

The subject of this present invention will be more clearly appreciated from the following description of the prefered embodiment taken in conjunction with the accompanying drawing in which
Fig. 1: longitudinal half-section of the bone bolt nut wrench.

The wrench has body 1 in the form of a bush. Square-shaped socket 2 is located at one side of body 1. The outer edge of socket 2 is ground off to give a point. At the other side of body 1 there is pin 10 connected to body 1 with screw 9. Pin 10 terminates in a handle. Inside of body 1 there is holder 3 connected to adjustment screw 4. Behind socket 2 there are two holes accommodating C-shaped spring part. Behind holder 3 there is nut 6.

The rear part of holder 3 has a tapped hole with adjustment screw 4 screwed therein. Adjustment screw 4 has tapped holes perpendicular to each other. Press pad 7 is screwed into the hole perpendicular to the longitudinal axis of adjustment screw 4 and screw 8, into the hole aligned with the longitudinal axis of adjustment screw 4.

Socket 2 of body 1 is used for mounting the nut and the bone bolt head. Holder 3 supports the part introduced into socket 2, by clamping spring part 5. Adjustment screw 4 which has been screwed into the tapped hole of holder 3 is prevented from unscrewing with nut 6. Press pad 7 is screwed into the hole drilled in adjustment screw 4 perpendicularly to the longitudinal axis through a longitudinal hole in the surface of body 1 and is prevented from unscrewing with screw 8.

Press pad 7 allows holder 3 to be moved inside 1. Opposite to sockets 2, pin 10 is screwed into body 1, used for mounting the handle and prevented from unscrewing with screw 9.

For the purpose of screwing in the bone bolt, holder 3 is moved backward with pressure pad 7. Next, the shank of the bolt is introduced into holder 3 so that the bolt head enters socket 2. Spring part 5 is clamped on the shank, thereby locking the entire bolt in body 1. After the bolt has been screwed into a bone, press pad 7 moves to its original position, thereby releasing the shank from clamped spring part 5.

For screwing the nut onto the threaded shank of the bolt, it is placed in socket 2 of body 1. Next, holder 3 is moved with press pad 7 so that it enters the hole of the nut. In the meantime, spring part 5 clamps on holder 3 locking it in the designed position.

When unscrewing the nut the wrench is applied to the nut with socket 2 having its edge ground off. The sharp edges of the socket degrease the tissue overgrowing the nut which sinks into socket 2 of body 1. Holder 3 connected to nut 6, adjustment screw 4, holder 7 and screw 8 move freely backwards. At the end of the nut unscrewing, press pad 7 is pressed and holder 3 is moved to the nut to engage the latter. After the nut has been unscrewed from the bolt shank, press pad 7 backs out to its original position and holder 3 moves out of the inside of the nut.

The bone bolt is unscrewed in the manner similar to that of its screwing in.

## Claims

1. A bolt nut wrench for bone anastomosis, having its body connected via a pin to a handle and press pad, characterized in that two holes are provided behind square socket (2) made in the face of said body (1) ground off on the external side, said two holes accommodating spring part (5) and said body (1) accommodating a holder (3) screwed with one end onto an adjustment screw (4) and with its other end entering said socket (2), a nut (6) being provided behind said holder (3) and a press pad (7) secured with a screw (8) being introduced behind said nut (6) into a hole of said body (1) and that of said adjustment screw (4).

10  9  8  7  4  6  3  1  2

5

0 272 647